Europäisches Patentamt

European Patent Office

Office européen des brevets

(11) Publication number: **0 350 715 B1**

## EUROPEAN PATENT SPECIFICATION

(45) Date of publication of patent specification: **30.11.94** (51) Int. Cl.⁵: **C12N 15/31**, C12P 21/02, A61K 39/02

(21) Application number: **89111747.5**

(22) Date of filing: **28.06.89**

(54) **Recombinant treponema hyodysenteriae antigen and uses therefor.**

(30) Priority: **29.06.88 US 213262**

(43) Date of publication of application:
**17.01.90 Bulletin 90/03**

(45) Publication of the grant of the patent:
**30.11.94 Bulletin 94/48**

(84) Designated Contracting States:
**AT BE CH DE ES FR GB GR IT LI LU NL SE**

(56) References cited:
**EP-A- 0 140 689**
**WO-A-88/04778**

**INFECTION AND IMMUNITY, vol. 56, no. 5, May 1988, pages 1070-1075, AmericanSociety for Microbiology, Washington, DC, US; S.N. CHATFIELD et al.:"Identification of the major antigens of Treponema hyodysenteriae and-comparison with those of Treponema innocens"**

(73) Proprietor: **ML TECHNOLOGY VENTURES, L.P.**
**1 Liberty Plaza**
**165 Broadway**
**New York New York 10080 (US)**

(72) Inventor: **McCaman, Michael**
**746 Cherry Avenue**
**San Bruno California (US)**
Inventor: **Dragon, Elizabeth**
**42 Park Lane**
**Orinda California (US)**
Inventor: **Gabe, Jeffrey D.**
**2364 Fulton**
**San Francisco California (US)**
Inventor: **Andrews, William H.**
**708 Fathom Drive**
**San Mateo California (US)**

(74) Representative: **Perry, Robert Edward et al**
**GILL JENNINGS & EVERY**
**Broadgate House**
**7 Eldon Street**
**London EC2M 7LH (GB)**

INFECTION AND IMMUNITY, vol. 54, no. 2, November 1986, pages 500-506, American-Society for Microbiology, Washington, DC, US; M.V. NORGARD et al.: "Cloning andex-pression of the major 47-kilodaton surface immunogen of Treponema pallidum in-Escherichia coli"

INFECTION AND IMMUNITY, vol. 57, no. 1, Januar 1989, pages 138-145, AmericanSocie-ty for Microbiology, Washington, DC, US; M. COENE et al.: "Comparativeanalysis of the genomes of intestinal spirochetes of human and animal origin"

## Description

Field of the Invention

This invention relates to vehicles expressing T. hyo. antigens that can be used in vaccines for swine dysentery.

Background of the Invention

Swine dysentery is a severe infectious disease found in all major pig-rearing countries. The symptoms of swine dysentery are severe mucohemoragic diarrhoea, dehydration and weight loss.

Swine dysentery is caused by Treponema hyodysenteriae, an anaerobic, B-hemolytic spirochete. The disease generally results from ingestion of faeces containing Treponema hyodysenteriae, from acutely-infected or asymptomatic carrier pigs or by faeces spread by farm equipment or handlers.

Chatfield et al, Infection and Immunity 56(5); 1070-1075 (May 1988), disclose the existence of several antigens of Treponoma hyodysenteriae.

Summary of the Invention

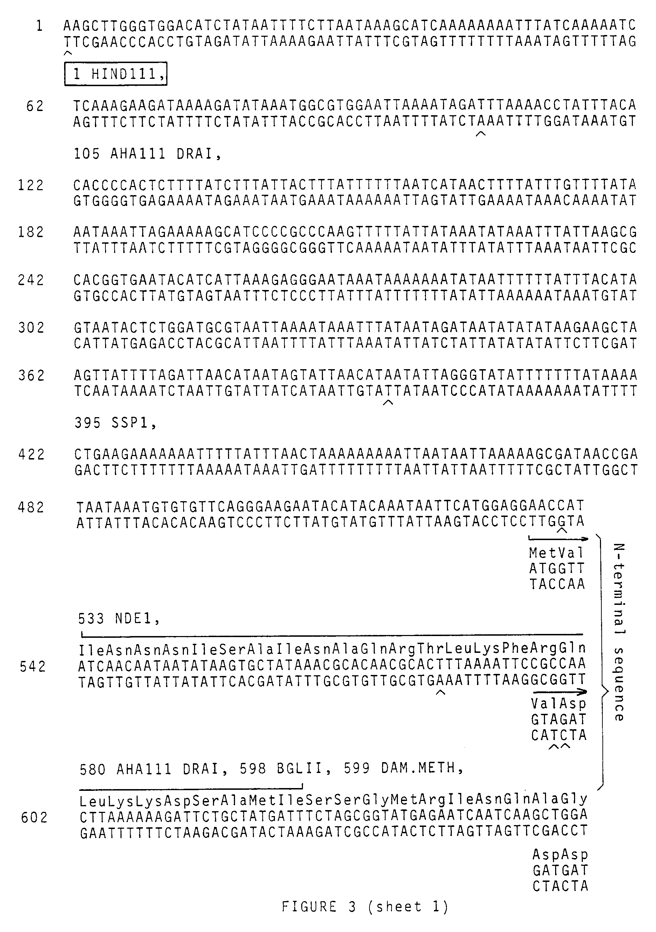

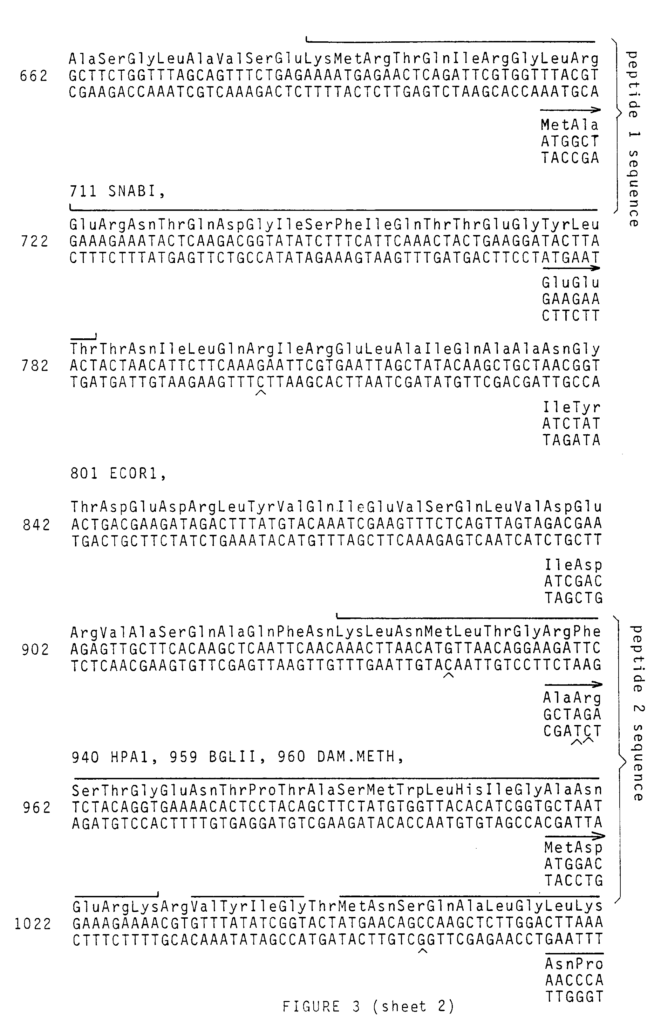

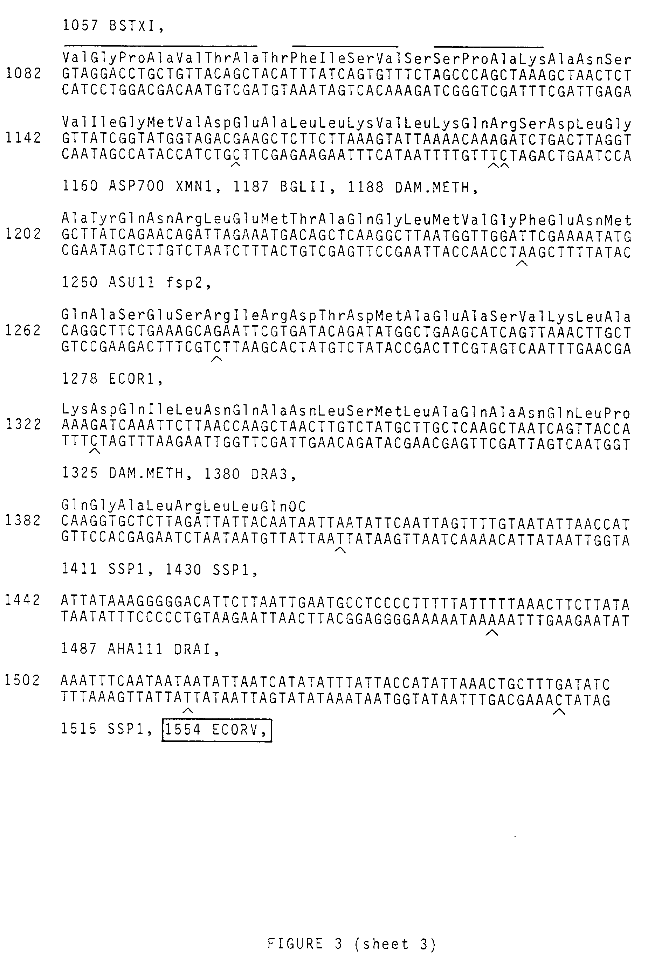

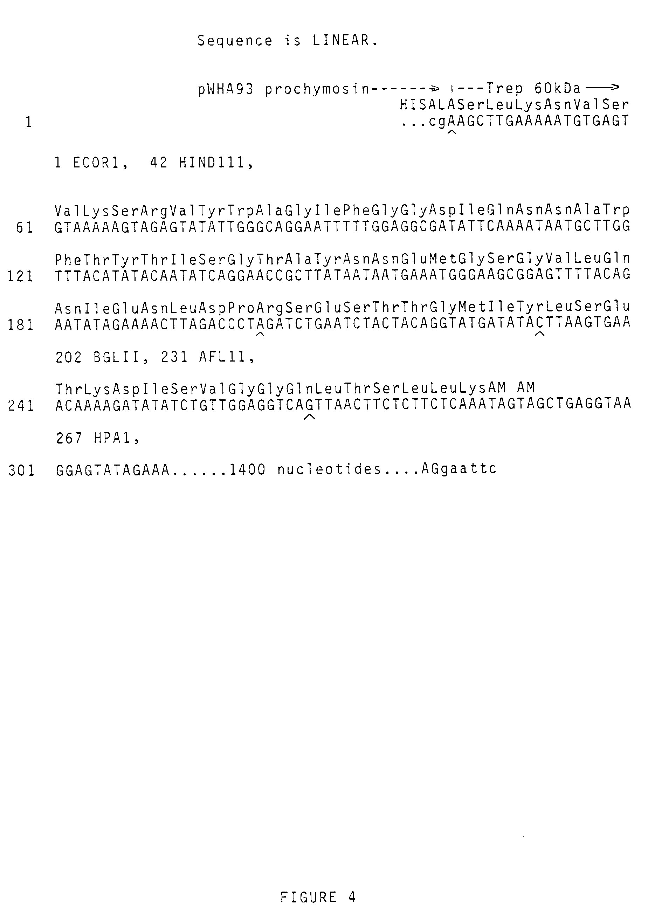

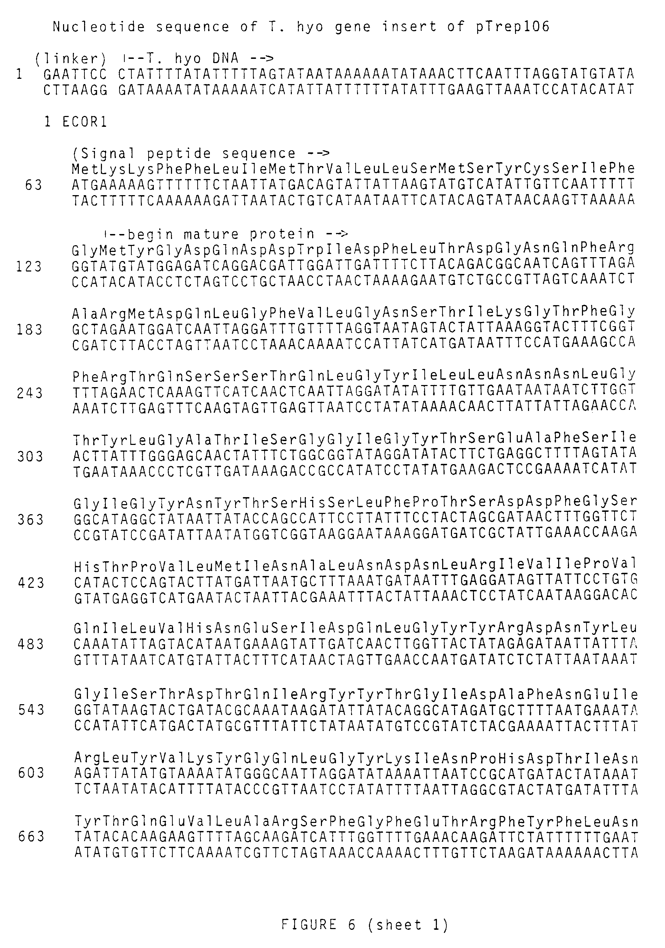

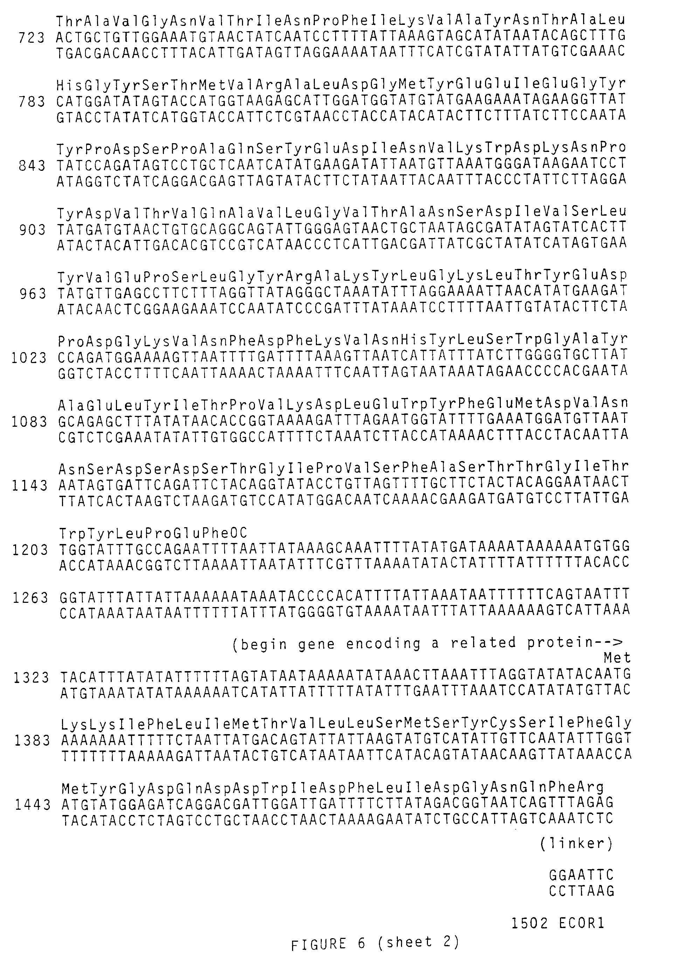

According to the present invention, novel expression vehicles include the DNA sequences shown in Figures 3, 4 and 6 or a fragment thereof which encodes a protein which is capable of eliciting an antibody which recognises, respectively, the 38 kDa, the 60 kDa or the 39 kDa T. hyo. antigen. In the accompanying drawings, Figures 3 and 6 respectively show the sequences of the 38 kDa and 39 kDa genes; Figure 4 shows the DNA and amino-acid sequence of the p Trep 103 (60 kDa-prochymosin) gene fusion.

These expression vehicles may be used to transform a host.

Description of the Invention

In the drawings (other than Figures 3, 4 and 6, described above):
Figure 1 is a restriction map of p Trep 2;
Figure 2 shows construction of the expression plasmid p Trep 9;
Figure 5 shows the construction of p Trep 103;
Figure 7 shows the DNA and amino-acid sequence of the p Trep 301 expression plasmid; and
Figure 8 shows a restriction map of p Trep 301.

The DNA sequence may encode for a protein which is a fusion product of (i) a protein which elicits antibodies which recognize an epitope(s) of the noted T. hyo. antigens end (ii) another protein (for example chymosin).

As a result, the term "DNA sequence which encodes for a protein which elicits antibodies which recognize an epitope(s) of the noted T. hyo. antigens" encompasses DNA sequences which encode for and/or express in appropriate transformed cells, proteins which may be the appropriate antigen, antigen fragment, antigen derivative or a fusion product of such antigen, antigen fragment or antigen derivative with another protein.

It is also to be understood that the DNA sequence present in the vector when introduced into a cell may express only a portion of the protein which is encoded by such DNA sequence, and such DNA sequence is within the noted terminology, provided that the protein portion expressed elicits antibodies which recognize an epitope(s) of one or more of the noted T. hyo. antigens. For example, the DNA sequence may encode for the entire antigen; however, the expressed protein is a fragment of the antigen.

The appropriate DNA sequence may be included in any of a wide variety of vectors or plasmids. Such vectors include chromosomal, nonchromosonal and synthetic DNA sequences; e.g., derivatives of SV40; bacterial plasmids; phage DNA's; yeast plasmids; vectors derived from combinations of plasmids and phage DNAs, viral DNA such as vaccinia, adenovirus, fowl pox virus, and pseudorabies.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda PL promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain a gene to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein. As representative examples of appropriate hosts, there may be mentioned: bacterial cells, such as E. coli, Salmonella typhimurium; fungal cells, such as yeast; animal cells such as CHO or Bowes melanoma; plant cells, etc.. The selection of an appropriate host is deemed to be within the scope of those skilled in the art from the teachings herein.

As hereinabove indicated, the expression vehicle including the appropriate DNA sequence inserted at the selected site may include a DNA or gene sequence which is not part of the gene coding for the protein which is capable of producing antibodies which recognize an epitope(s) of the noted T. hyo. antigen(s). For example, the desired DNA sequence may be fused in the same reading frame to a DNA sequence which aids in expression or improves purification or permits expression of the appropriate protein. For example, as shown in representative embodiments of the present invention, the appropriate DNA sequence is inserted in a vector in the same reading frame as a gene which encodes for calf chymosin, and the desired protein is expressed as a fusion peptide with calf chymosin.

When seeking to develop a vaccine neutralizing or protective antibodies could be targeted towards discontinuous, conformation-dependent epitopes of the native antigen. One must therefore consider whether the protein obtained from the recombinant expression system might have a three dimensional structure (conformation) which differs substantially from that of the original protein molecule in its natural environment. Thus, dependent on the immunogenic properties of the isolated proteins, one might need to renature it to restore the appropriate molecular conformation. Numerous methods for renaturation of proteins can be found in the scientific literature and include; (1) denaturation (unfolding) of improperly folded proteins using agents such as alkali, chaotropes, organic solvents and ionic detergents followed by a renaturation step achieved by dilution, dialysis, or pH adjustment to remove the denaturant, and (2) reconstitution of proteins into a lipid bilayer or liposome to re-create a membrane like environment for the immunogenic protein.

In accordance with another aspect of the present invention, the protein produced from a host transformed with a cloning vehicle of the type hereinabove described may be employed in conjunction with a physiologically acceptable vehicle to provide protection against swine dysentery, and in particular swine dysentery induced by T. hyo.. As hereinabove indicated, such protein is capable of eliciting antibodies which recognize an epitope(s) of one or more of the hereinabove noted T. hyo. antigens. Such expressed protein will be sometimes hereinafter referred to as a "recombinant T. hyo. antigen," however, as hereinabove indicated, such protein may not correspond to a T. hyo. antigen in that it may also be a fragment, derivative or fusion product. The term "recombinant T. hyo. antigen" also encompasses such fragments, derivatives and fusion products.

The recombinant T. hyo. antigen is employed in the vaccine in an amount effective to provide protection against swine dysentery. In general, each dose of the vaccine contains at least 5 micrograms and preferably at least 100 micrograms of such recombinant T. hyo. antigen. In most cases, the vaccine does not include such recombinant T. hyo. antigen in an amount greater than 20 milligrams.

The term "protection" or "protecting" when used with respect to the vaccine for swine dysentery described herein means that the vaccine prevents swine dysentery and/or reduces the severity of swine dysentery.

If multiple doses are given, in general, they would not exceed 3 doses over a six week period.

The vehicle which is employed in conjunction with the recombinant T. hyo. antigen may be any one of a wide variety of vehicles. As representative examples of suitable carriers, there may be mentioned: mineral oil, alum, synthetic polymers, etc. vehicles for vaccines are well known in the art and the selection of a suitable vehicle is deemed to be within the scope of those skilled in the art from the teachings herein. The selection of a suitable vehicle is also dependent upon the manner in which the vaccine is to be administered. The vaccine may be in the form of an injectable dose and may be administered intramuscularly, intravenously, or by sub-cutaneous administration. It is also possible to administer the vaccine orally by mixing the active components with feed or water; providing a tablet form, etc.

Other means for administering the vaccine should be apparent to those skilled in the art from the teachings herein; accordingly, the scope of the invention is not limited to a particular delivery form.

It is also to be understood that the vaccine may include active components or adjuvants in addition to the recombinant T. hyo. antigen or fragments thereof hereinabove described.

4

The present invention will be further described with respect to the following examples; however, the scope of the invention is not to be limited thereby. In the Examples, unless otherwise noted, purifications, digestions and ligations are accomplished as described in "Molecular Cloning, a laboratory manual" by Maniatis et al. Cold Spring Harbor Laboratory (1982). In the following examples, unless otherwise indicted, transformations are accomplished by the procedure of Cohen et al. PNAS 69 2110 (1973).

EXAMPLE 1

FERMENTATION

The virulent T. hyo. strain B204 was cultured anaerobically (90% N2, 10% $CO_2$ atmosphere) in a 2 liter fermenter in which a pH of 6.8 and a temperature of 37°C was maintained. The culture medium was comprised of Brain-Heart Infusion, 5% fetal calf serum, dextrose, and spectinomycin dihydrochloride.

The culture was harvested at a cell density of approximately 5 x $10^8$/ml. The cells were collected by centrifugation, then washed in 10mM acetate buffer pH 4.75 containing 150mM KCl and recentrifuged. The wash procedure was repeated twice more and the final cell pellet was stored at -20°C.

PROTEIN PURIFICATION

Frozen cells were thawed and then resuspended in 10mM potassium acetate pH 4.75 until an optical density of 25-30 (at 600mm) was achieved (as measured on solution dilutions) which is typically 1/20 the original culture volume. Tween®-20 (a non-ionic detergent) was then added to the cell suspension to achieve a final concentration of 0.2%. After gentle agitation for 10 minutes the cells were centrifuged (10,000 x g for 10 min). This supernatant fraction was discarded and the cells were resuspended in acetate buffer and then extracted with 2.0% Tween®-20. After centrifugation the 2% Tween® supernatant (detergent solubilized antigen pool) was saved and the cell pellet was resuspended and re-extracted with Tween-20 in a sequential manner for up to 3 additional cycles with the concentration increasing over the cycles from about 2% up to about 10%. The detergent solubilized supernatant fractions were pooled. This extraction procedure selectively (but not quantitatively) solubilizes surface proteins of T. hyo. without lysing or rupturing the bacteria.

After high speed centrifugation at 100,000 x g the pellet from Tween® 20 solubilized proteins was resuspended by sonication in 1/500th original culture volume of 25mM Tris-Cl, pH 6.8 (total protein concentration of 20 mg/ml). This suspension was then mixed with 3/100th of the original culture volume of the same buffer containing 6M urea for 1 hour at room temperature with gentle agitation. This step solubilizes approximately 80% of the protein in the original pellet. The urea insoluble proteins (UPI) were collected by ultracentrifugation at 100K x g for 90 minutes at 4°C, resuspended in 1/500th the original culture. volume of 25mM Tris-Cl, pH 6.8 and extracted a second time with 3/100th's of the original culture volume of the same buffer containing 6M urea for 1 hour at room temperature with gentle agitation. This second urea extraction selectively solubilizes a 38Kd protein and the 29Kd which is a minor component of UP2. Other proteins not solubilized by the second urea extraction were removed by ultracentrifugation at 100K x g for 90 minutes at 4°C. The supernatant from the second urea solubilization (US2) was dialyzed against 10mM $NH_4HCO_3$ to remove urea and then lyophilized. This material was then solubilized in 25mM Tris-Cl pH 6.8 and stored at -20°C. Starting with 10 gms wet weight of T. hyo. cell paste (1.5 liter culture), there is obtained 4mg of the 38kD protein and 0.4 mg of the 29kD protein in the US2 fraction.

AMINO ACID SEQUENCING OF THE 38kD PROTEIN

The amino acid sequence of the amino terminus of the 38 kDa protein/ was determined using sequential Edman degradation in an automated Applied Biosystems® gas phase protein sequencer. The released amino acids from the 38kD protein were derivitized with phenylisothiocyanate (PITC) and then separated and analyzed by HPLC. The identity of the first 30 amino acids of the protein, thus determined, are shown below:

2                10

?-Val-Ile-Asn-Asn-Asn-Ile-Ser-Ala-Ile-Asn-Ala-Gln-Arg-Thr-

20                30

Leu-Lys-Phe-Arg-Gln-Val-Asp-Leu-Lys-?-Asp-?-Ala-Met-Ile-

Additional amino acid sequence from the 38 kDa protein was obtained by digesting 380$\mu$g of US2 (1 nanomole) with 8$\mu$g of endoproteinase Lys C for 4 hrs at 37°C in ll3$\mu$l of 50 mM Tris-Cl pH 6.8. The peptide fragments obtained from this digest were separated by HPLC on a C4 column using a gradient of 0-100% acetonitrile: isopropanol (2:1) in 0.1% trifluoroacetic acid. Absorbance was monitored at 214nm and 280nm. Fractions containing major peaks were lyophilized, resuspended in 0.1% SDS and sequenced as above. Amino acid sequence was obtained from 2 internal proteolytic fragments of the 38 kDa protein and are shown below:

1                10

(peptide 1) Met-Arg-Thr-?-Ile-Arg-Gly-Leu-Arg-Met-Ala-Glu-Arg-Asn-

20

Thr-?-Asp-Gly-Ile-?-Phe-Ile-?-Thr-Glu-?-Gly-Tyr-

30

Leu-Glu-Glu-Thr-

1                10

(peptide 2) Leu-Asn-Met-Leu-?-Gly-Arg-Phe-Ala-Arg-Ser-Thr-Gly-Glu-

20

Asn-Thr-Pro-Thr-Ala-Ser-?-Trp-Leu-His-Ile-Gly-Ala-Asn-

30

Met-Asp-Glu-Arg-Lys-

## PREPARATION OF T. hyo DNA

A 1 liter culture of T. hyo strain B204 was centrifuged and the bacterial cells resuspended in 6ml phosphate buffered saline (this brought the total volume to 9.5 ml). This was then mixed with 10 ml of Solution A (113.6 gr CsCl, 87.6 ml TE buffer (10mM Tris/1 mM EDTA pH 8.0)), and 6.88 ml 20% Sarkosyl®, a detergent, was added to lyse the cells. Then 3.5 ml of TE buffer and 6.12 ml 10 mg/ml EtBr were added and the solution was spun in a Sorvall® TV850 rotor at 43,000 rpm overnight. The band of genomic DNA was withdrawn with an 18 gauge syringe, mixed with 50% (w/w) CsCl and spun in a Sorvall® TV865 rotor for 7 hours at 55,000 rpm. The genomic DNA was withdrawn again and spun in CsC1 in TV865 rotor overnight. After withdrawing the genomic DNA the ethidium bromide was removed by extraction with isopropanol. The DNA was then extensively dialyzed against TE and stored at -20°C.

## Oligonucleotide Probe

A partial amino acid sequence had been described above for the T. hyo 38 kDa antigen. All possible DNA sequences that could encode the first six amino acid region of the N-terminus of the protein were determined and a mixed probe was generated. This mixed probe is a pool of sequences which encompass all the possible combinations of nucleotides which could encode the amino acid sequence of the target region as indicated below. Such a mixed probe is called degenerate. Each probe is 17 nucleotides in length. Thus, this probe, COD450, is 96 fold degenerate. The COD450 was made on a DNA synthesizer.

```
                              COD450

              Val-Ile-Asn-Asn-Asn-Ile

                  C

                  A    C

                  G    A    C    C    C

              GTT ATT ATT ATT ATT AT
```

### T. hyo DNA Analysis

T. hyo strain B204 DNA was digested with Hind III and run on a 1% Agarose gel. The gel was then blotted according to Southern (JMB 98:503) and probed using COD450. Hybridization conditions were as follows:

The dried filter was rewetted in 0.9 M NaCl, 90 mM Tris-HCl, pH 7.5, 6 mM EDTA. The filters were then placed into bags and 2.5 ml pre-hybridization solution filter was added.

The prehybridization/hybridization solution contained:

6X NET
5X Denhardts
0.1 mM rATP
1.0 mM NaPPi
10.0 mM Sodium Phosphate pH 7.5
0.2% SDS
0.1 mg/ml sonicated salmon sperm DNA
250 $\mu$g/ml E. coli tRNA

Prehybridization was done at 37°C for 2 1/2 hours.

The pre-hybridization solution was then drained from the bags and 3 ml of a second pre-hybridization solution was added to each bag. The second prehybridization solution was identical to the first except that 1 ml of 100 mg/ml lambda gtII DNA and 5 ml of 362 $\mu$g/ml E. coli DNA was added per 50 ml. Both DNAs were sonicated to less than 500 bp, boiled 10 min and quick chilled immediately prior to use. The filters were allowed to pre-hybridize in this solution for 2 1/2 hours at 37°C.

Probes were kinased as follows: 6.24$\mu$l of COD450 (1.57 OD/ml) using 37 $\mu$l P$^{32}$-ATP (7000 Ci/mMole) in an 80 $\mu$l reaction mix with 20 units of BMB kinase.

The kinased probe, COD450, was added to 30 ml prehyb solution. The bags were drained of prehybridization solution and the probe containing hybridization solution was added. These were allowed to incubate overnight (16 hours) at 37°C.

The filter was then washed 4 X 5 min in 1 liter 6X SSC at room temp followed by 4 X 5 min in 100 ml 3 M Tetramethylammonium chloride, 50 mM Tris-HCl, pH8.0, 2 mM EDTA, 1 mg/ml SDS (TMAC) (DNA, 82, 1585-1588) at room temp. The filter was then washed at 55°C for 5 min in 400 ml TMAC. The filter was then wrapped in plastic wrap (without first drying) and exposed to Kodak® XAR5 film for 1-1/2 hours with double intensifying screens at -80°C.

Analysis of the autoradiograph showed that two DNA fragments hybridized to COD450, they were 3200 bp and 2300 bp in size.

The Southern blot was removed from the plastic wrap and was washed once in 400 ml TMAC for 5 min at 60°C. This was then re-exposed to XAR5 film as above and the autoradiograph showed that COD450 had melted off of both the 3200 and 2300 bp DNA fragments.

Preparation of Target DNA

145.6 μg genomic DNA of T. hyo B204 was digested with Hind III (BMB). 80 μg of this DNA was electrophoresed through a 3% acrylamide gel. Using lambda DNA, digested with Hind III, as a size marker, everything in the preparative T. hyo digest between 2 Kb and 3.5 Kb was cut out of the gel in 0.5 cm slices. This resulted in a total of 7 gel slices each containing a different size class of Hind III fragments. Each size fraction was electroeluted, phenol extracted, ethanol precipitated and resuspended in 50 μl of water.

5 μl of each fraction was mixed with 200 μl 10 mM Tris-HCl pH 7.4, 20μl 3M NaOH and incubated at 65°C for 30 min. The samples were then allowed to cool to room temp for 15 min and 200 μl 2M NH₄OAC was added. These samples were then slot blotted onto nitrocellulose using the apparatus and protocol prescribed by Schleicher and Schuell. The slot blot filter was then re-wetted in 6X NET and placed in a plastic bag. Five ml of prehybridization solution was added as before only the solution was made up without E. coli tRNA. The filter was incubated for 5 hours at 37°. More COD450 was kinased as described above. The slot blot filter was hybridized, washed, and exposed to film in the same way as the southern blot was described above.

Examination of the autoradiograph showed that fraction five gave the strongest hybridization signal. A sample of fraction five DNA was sized on an agarose gel at about 2300-2700 bp. Fraction five was chosen as the fraction to be cloned.

0.035μg of Fraction 5 DNA was ligated to 0.017μg of Hind III digested pUC9 and transformed into DH5 competent cells (BRL). The transformation was plated on plates containing 50 μg/ml X-gal. The resulting 206 white colonies were all cultured and miniscreened.

Aliquots of all 206 miniscreens were digested with Hind III and hybridized to Cod450 by slot blot analysis as described above. Two positive clones were identified, pTrep2 and pTrep3.

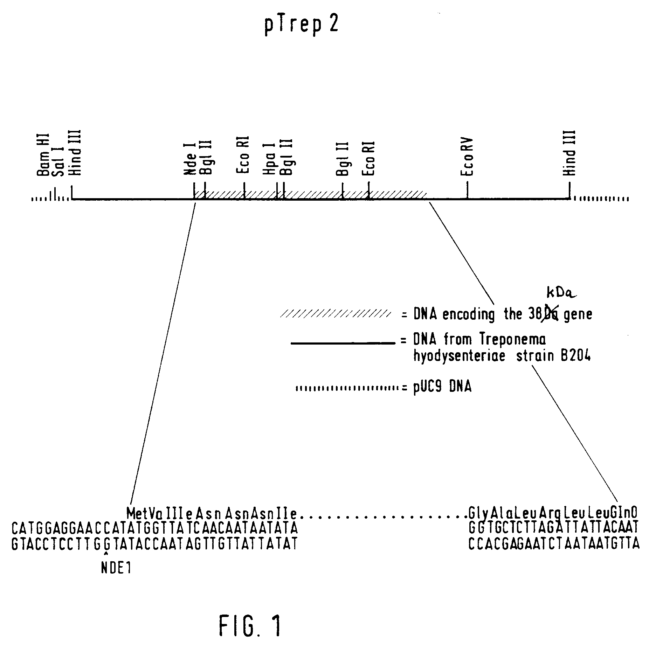

Plasmid DNA from pTrep2 was digested with 30 different restriction enzymes for restriction mapping. The digests were analyzed by Southern blotting against Cod450. The resulting restriction map is depicted in Fig. 1.

Expression of the 38 kDa Antigen in E. coli

The 38 kDa antigen was to be expressed as a fusion to prochymosin using the vector pWHA93.
The 38 kDa antigen was to be expressed as a fusion to prochymosin using the vector pWHA93.

First it was necessary to invert the Hind III insert of pTrep2 to make best use of available restriction sites. PTrep2 was Hind III digested, ligated, transformed into E. coli JM83 and a clone, pTrep7, was identified which carried the 38 Kd gene on the Hind III fragment in the reverse orientation from pTrep2.

15 μg of pTrep7 was digested with Nde I and treated with T4 DNA polymerase (BRL) to fill in the 2 base Nde I overhang. The DNA was phenol extracted, ethanol precipitated and resuspended in the appropriate buffer for Hind III digestion. Following Hind III digestion, the DNA was electrophoresed through a 5% acrylamide gel. The 1900 bp Hind III - Nde I fragment was cut out of the gel and electroeluted.

The expression vector, pWHA93, was digested with Hind III and Hinc II run on a gel and the 3870 bp fragment was electroeluted. Aliquots of the T. hyo fragment and the pWHA93 vector were ligated and transformed into JM83 according to Kushner, S.R. in "Genetic Engineering" - Elsevier/North Holland BioMedical Press 1978 p. 17-23.

From this transformation pTrep9 was identified by miniscreen analysis. This plasmid was sequenced and found to have the correct fusion to place the 38 kDa open reading frame in the same phase as and downstream of the prochymosin open reading frame.

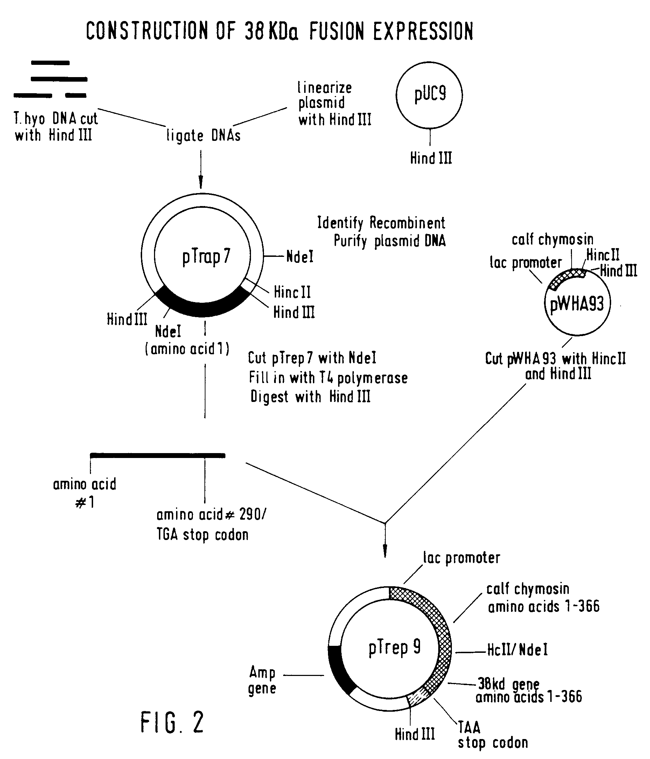

The sequence for production of pTrep9 is schematically illustrated in Figure 2.

The expression and recovery of protein was effected as hereinafter described with respect to the 39 kDa antigen (Example 2).

In E Coli pTrep9 was observed to direct the synthesis of a 73 kDa prochymosin fusion polypeptide which is 32 kDa larger than prochymosin. By sequence analysis, the open reading frame of the 38 kd antigen gene appears to encode a 32 kDa polypeptide.

This fusion protein showed immunoreactivity equivalent to the authentic T. hyo 38 kDa protein when reacted in Western blots with sera from pigs recovered from swine dysentary or sera from mice hyper-immunized with the purified 38 kDa T. hyo protein.

The portion of the amino acid sequence which has been overscored has been verified by peptide sequencing.

EP 0 350 715 B1

### Preparation of pWHA93

pWHA93 was constructed through a series of steps. Plasmid pWHA3IB (J. Biotech, 2, 177-190, 1986), which contains the entire coding sequence for prochymosin, was digested with the restriction endonuclease BclI. The prochymosin gene stop codon, TGA, is contained within the nucleotide sequence of the BclI restriction endonuclease site: TGATCA. Mung bean nuclease (New England Biolabs) was then used to make the BclI generated ends blunt. This step was followed by digestion with EcoRI. The 618 base pair (bp) DNA fragment that contains the coding sequence for the carboxyl terminal 205 amino acids was then isolated.

pUCl8 (New England Biolabs) was digested with restriction endonuclease KpnI, treated with mung bean nuclease, digested with EcoRI, and the 2674 bp DNA fragment was isolated. This fragment contains a beta-lactamase selectable marker (AMP$^r$ gene), an origin of replication, the lactose operon (lac) promoter, a polylinker containing sites for the restriction endonucleases BamHI, XbaI, SalI, PstI, SphI, and HindIII, and the coding sequence for the amino terminal 82 amino acids of beta-galactosidase.

The 2674 bp DNA fragment was ligated to the 618 bp DNA fragment to form pMH22. This plasmid contains the coding sequence for the carboxyl terminus of prochymosin fused to one end of the polylinker while the other end is fused to the coding sequence for the amino terminus of beta-galactosidase. The prochymosin stop codon, TGA, was destroyed with the mung bean nuclease and the two coding sequences are in the same translational reading frame.

pWHA43 (J. Biotech., 2, 177-190, 1986) was also used to construct pWHA93. This plasmid was digested with EcoRI, treated with mung bean nuclease, and digested with the restriction endonuclease HindIII. The 3654 bp DNA fragment containing all of the coding sequence for prochymosin, the Amp$^r$ gene, an origin of replication, and the lac promoter was isolated.

pDR540 (P-LBiochemicals) was digested with the restriction endonuclease BamHI treated with mung bean nuclease, and digested with HindIII. The 92 bp DNA fragment containing the Tac promoter was isolated and ligated to the 3654 bp DNA fragment to construct pWHA49. This plasmid has the entire coding sequence for prochymosin under the control of the tandem Lac and Tac promoters.

pWHA49 was digested with restriction endonucleases NarI and EcoRI. The 3049 bp DNA fragment containing the coding sequence for the amino terminal 160amino acids of prochymosin, the Lac and Tac promoters, the Amp$^r$ gene, and an origin of replication was isolated.

pMH22 was digested with NarI and EcoRI and the 200 bp DNA fragment containing the coding sequence for the carboxyl terminal 205 amino acids of prochymosin, the polylinker and the coding sequence for the amino terminal 52 amino acids of beta-galactosidase was isolated. This 200 bp DNA fragment was ligated to the 3049 bp DNA fragment to construct pWHA93 which contains the entire prochymosin coding sequence, the polylinker, and the coding sequence for the amino terminal 82 amino acids, fused together in phase, under the control of the lac and tac promoters. It was later discovered that the tac promoter had been deleted from pWHA93 by site specific recombination leaving an intact lac promoter.

### Example 2 - 39 kDa antigen

The fermentation and protein purification are accomplished as in Example 1.

The insoluble material obtained by centrifugation of the second urea extraction contains a single major protein component which is 39 kDa. This insoluble protein was solubilized by boiling in 25 mM Tris-HC1 pH6.8 containing 3%SDS, 1 mM EDTA, and 70 mM 2-mercaptoethanol. This solution was subjected to gel filtration chromatography over a 30 cm column of Sepharose® 6B (from BioRad, Richmond, CA). The 39 kDa peak was identified by gel electrophoresis of column eluant fractions, the appropriate fractions were pooled and the protein concentrated by precipitation with acetone and collected by centrifugation. The pellet was dissolved in 1.1% SDS and then with chloroform/methanol to remove residual SDS.

The amino acid sequence of the amino-terminus of the 39 kDa protein prepared above was determined using sequential Edman degradation in an automated Applied Biosystems gas phase sequenator. The identity of the first 41 amino acids of the protein thus determined are shown below:

$$1 \qquad\qquad\qquad 10$$

Met-Tyr-Gly-Asp-Arg-Asp-Ser-Trp-Ile-Asp-Phe-Leu-Thr-His-Gly-

$$20$$

Asn-Gln-Phe-Arg-Ala-Arg-Met-Asp-Gln-Leu-Gly-Phe-Val-Leu-Gly-

$$41$$

Asn-Asp-Thr-Ile-Lys-Gly-Thr-Phe-?-?-Arg-

## OLIGONUCLEOTIDE PROBE DESIGN

A set of DNA probes were synthesized using the amino sequence data shown above. Each of them were comprised of a pool of degenerate sequences which encompass all the possible combinations of nucleotides which could encode the amino acid sequence of the target region as indicated below. Each probe is 17 nucleotides in length.

```
      1

Met-Tyr-Gly-Asp-Arg-Asp        probe name = COD 555
ATG-TAT-GGT-GAT-AGT-GA
        C   C   C   C
            A   .   A          degeneracy = 128 fold
            G   ·   G            (mix of 128 combinations)

      10

Trp-Ile-Asp-Phe-Leu-Thr        probe name = COD 553
TGG-ATT-GAT-TTT-TTT-AC
        C   C   C   C
        A           A
                    G          degeneracy = 96 fold

      18

His-Gly-Asn-Gln-Phe-Arg        probe name = COD 556
CAT-GGT-AAT-CAA-TTT-AG
    C   C   C   G   C C
            A                  degeneracy = 128 fold
            G
```

## CONSTRUCTION OF A GENOMIC LIBRARY OF T. hyo DNA

48 $\mu$g Genomic DNA of T. hyo strain b204 was digested with 6 units AluI. Before adding the enzyme the solution was incubated at 37°C for 10 minutes. The reaction conditions were those prescribed by BRL and the total volume was 480 $\mu$l. After addition of the enzyme the reaction was incubated at 37°C. 96 $\mu$l aliquots were removed at 15 sec, 2 min 11 sec, 4 min 8 sec, 6 min 4 sec, 8 min after addition of the enzyme. Each aliquot was immediately mized with 96 $\mu$l phenol-CHCl$_3$ 4 $\mu$l was run on agarose gel. Visualization of the digested DNA s under UV light showed that the reactions had proceeded as far as was predicted from previous analytical digests. The remainder of the aqueous phases were combined to obtain one solution 460$\mu$l which was extracted phenol/-CHCl$_3$, re-extracted with chloroform, and the DNA precipitated with Ammonium acetate EtOH. The DNA was then resuspended to a concentration of 200 $\mu$g/ml.

### III. Linker addition

Phosphorylated EcoRl Linkers: 5' pd [GGAATTCC] 3' were obtained from Pharmacia and were diluted to 1 mg/ml in $H_2O$. 0.2 $\mu$g of the linker was enzymatically kinased with $P^{32}$-ATP by standard procedures. Following the kinase treatment, this reaction was incubated at 90°C for 10 minutes, evaporated to dryness in a rotovac evaporator and resuspended in 20 $\mu$L of 1 mg/ml non-radioactivity labeled phosphorylated EcoR1 linker.

The EcoR1 linker mixture was then ligated to the AluI partially digested T. hyo DNA (from above) at a linker concentration of 100 $\mu$g/ml and a T. hyo DNA concentration of 133 $\mu$g/ml using BMB ligase at a concentration of 50 units/ml. The total volume of the reaction was 150 $\mu$l. The ligation was allowed to incubate overnight ( 16 hours) at room temp and then boosted with 3$\mu$l of 1 unit/$\mu$l ligase. This was incubated 2 hours at room temperature. The reaction was then boosted again by addition of 5 $\mu$l fresh 10 mM rATP and 3 $\mu$l ligase and incubated for 2 hours at room temp. The ligase was then heat inactivated. Following this, the ligation mix was digested with EcoR1 500 units/ml for 2 hours at 37°C. (Note: Treponema hyodysentariae DNA is resistant to EcoR1 digestion presumably because the EcoR1 sites are methylated). The reaction was then phenol extracted and ethanol precipitated. The precipitated DNA was resuspended in 100 $\mu$l $H_2O$.

The DNA was fractionated on an S-200 (Pharmacia) column using 0.3 M NaCl, 0.05 m Tris-HCl pH8.0, 1 mM EDTA, 0.06% sodium azide as a column buffer. Eighty 100 $\mu$l aliquots were collected and counted in a scintillation counter. The first peak was observed in fractions 21-33 (T. hyo DNA) and the second peak was observed in fractions 38-80 (free linkers and free ATP). Fractions 21-33 were pooled and precipitated with ethanol. The DNA was resuspended in 40.35 $\mu$l $H_2O$ to bring the concentration to 200 $\mu$g/ml.

Dephosphorylated lambda gtll EcoRl arms were obtained from Promega Biotec at a concentration of 500 $\mu$g/ml. The T. hyo DNA was ligated to lambda at a T. hyo DNA concentration of 80 $\mu$g/ml and a lambda gtll concentration of 200 $\mu$g/ml in a total volume of 6.25 $\mu$l using BMB ligase at a concentration of 100 units/ml. The ligation was allowed to incubate at room temperature for 2 hours. 4 $\mu$l of the ligation was then packaged into lambda bacteriophage particles using the in vitro packaging kit, "Gigapack®," obtained from Stratagene (San Diego, CA). The phage was then titered on a stationary phage culture of E. coli strain Y1090r-(Promega Biotec). The number of plaques, blue (non-recombinants) and white (recombinant) were counted and indicated that the original phage stock contained 1.4 X 10$^7$ pfu/ml in a total of 0.5 ml (in which 91% were recombinants).

The gene library was characterized by miniscreening twelve randomly selected white plaques (Helms, C. et al, DNA 4 39-49 (1985)). All twelve had inserts, with an average insert size of 1,325 bp.

### IDENTIFICATION OF A RECOMBINANT PHAGE

In performing mixed oligonucleotide screening fo the 39K gene, the following procedures were used:
1. The lambda gt11 (AluI partial) library was used. Approximately 10,000 plaques per 150 mm petri dish were plated on Y1090r-cells. Six plates were used and four nitrocellulose filters were lifted off of each plate (procedure of W. D. Benton & R.W. Davis Science 196, 180 (1977).
2. The mixed oligonucleotide probes were kinased to a specific activity of 4-10 X 10$^5$ cpm/ng. Duplicate filters were hybridized with each probe; each filter was probed with approx. 10$^6$ cpm (1-2 ng probe/filter).
3. The hybridization solution consisted of:
5X Denharts
0.1 $\mu$m rATP
250 $\mu$g/ml E. coli tRNA
6X NET (1XNET = 0.15 M NaC1, 15 mM Tris-HCl pH 7.5, 1 mM EDTA)
0.5% NP40
1 mM sodium pyrophosphate
4. Filters (as in 1 above) were pretreated (prior to hybridization) as follows:
   1. Prewashed 2 hrs at 37°C in: 50 mM Tris-HCl, pH 8, 1M NaCl, 1mM EDTA, 0.1% SDS
   2. Prehybridization: 2 hrs at 37°C in Hyb Buffer (as in 3 above).
5. Hybridization overnight at 37°C.
6. Filter washing following hybridization: Filters were washed twoice at RT (20 min/wash) in 6X NET, 0.1% SDS (2 L/wash) Twice (20 min/wash) at 37°C in 6X NET, 0.1% SDS Once (20 min/wash) at 37°C in 6X NET
7. Filters were then air dried, taped to backing and exposed to Kodak® XAR5 X-ray film (using 2 intensifying screens).

EP 0 350 715 B1

8. Appropriate plaques showing hybridization to both probes were selected, picked and rescreened with both oligonuc. probes.

9. Following 3 rounds of plaque purification, phage 3-5C1 was selected as the protoype 39K recombinant phage.

10. Hybridization of probes to genomic T. hyo DNA, phage DNA, and or plasmid DMA was done according to the procedure of Southern (J.Mol.Biol. 94, 203 [1975]).

11. Phage DNA was isolated using the Technique of C. Helms, et al. (DNA 4, 39-49, [1985]). This DNA was examined following digestion with Eco R1 and found to contain 1 6 kb insert which hybridized with oligonucleotides COD 553 and 555 and thus contained the desired T. hyo DNA which should encode the 39 kDa treponemal antigen.

SUBCLONING AND EXPRESSION OF THE GENE FOR THE 39 kDa ANTIGEN

1. The Eco R1 flanked, 1.6 kb segment of DNA from phage 3-5C1 was isolated by electroelution from an acrylamide gel and then ligated to plasmid pUC 19 which had been linearized by digestion with EcoR1. These DNAs were the ligated together, transformed into E. coli, and a clone containing recombinant plasmid pTREP 106 (Figure 6) was identified by analysis or restriction digests of plasmid DNA.

2. Plasmid pTREP 106 was used to direct protein synthesis in an in vitro coupled transcription-translation system containing $^{35}$S-Methionine. SDS-gel electrophoresis of the protein products of this system showed at 39 kDa protein species not seen with the parental plasmid lacking the T. hyo DNA insert. This suggests that the cloned DNA contains the complete coding sequence for the T. hyo 39 kDa antigen and that E. coli is capable of recognizing the treponemal promoter and ribosome binding site and directing the synthesis of this foreign protein.

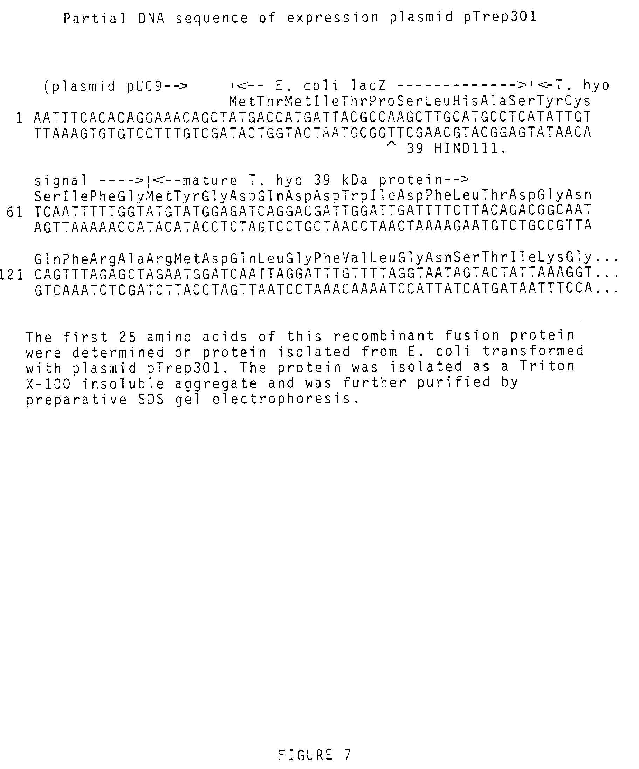

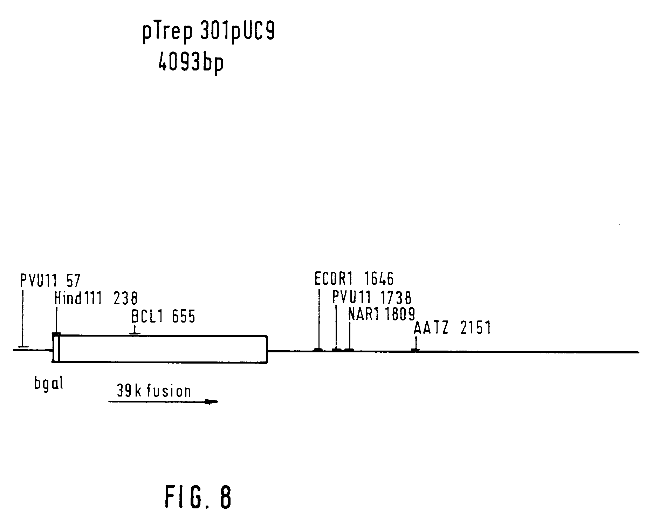

3. E. coli. strains transformed with plasmid pTrep 106 did not produce significant amounts of the desired 39 kDa T. hyo. antigen. Therefore, plasmid construction allowing high level expression of the recombinant antigen was made as follows. The Eco RI flanked, 1.6 kb fragment of pTrep 106 was ligated to plasmid pUC 18 linearized by digestion with Eco RI. The resulting plasmid, pTrep 112, was then cut with PstI and BamHI, then treated with exonuclease III to remove (in a unidirectional manner) the non-coding DNA sequence upstream of the predicted ATG start codon of the 39 kDa T. hyo. antigen. At various times during this digestion, DNA aliquots were removed the exo III inactivated by phenol extraction, the remaining DNA rendered blunt ended by digestion with nuclease S1, and this DNA was then religated and used to transform E. coli. Nucleotide sequencing of plasmid DNA from one such new clone, pTrep 112-1, indicated that a contiguous sequence of 372 codons encoding the mature T. hyo. 39 kDa protein and 7 amino acids from the signal sequence were fused downstream of the Hind III site of the parental pUC 18 plasmid. The fusion was in a reading frame to encode a fusion protein whose expression would be regulated by this lac promoter after the orientation of the cloned fragment was inverted by cloning into pUC 9 from the HindIII to Eco RI site. E. coli. transformed with the resulting plasmid, Trep 301, (Figures 7 and 8) produced an insoluble 40 kDa antigen which reacts with sera from swine (both those recovered from S.D. as well as animals immunized with the 39 kDa protein purified from T. hyo.) in both an immunoblot and plate ELISA assay.

EXPRESSION OF THE RECOMBINANT FORM OF THE 39 KDA ANTIGEN

E. coli strain JM83 containing plasmid pTrep301 was grown in 250 mls of Luria broth containing ampicillin at 200 µg/ml. The culture was grown for 18 hours at 32-37 degrees C. The cells were harvested by centrifugation then resuspended in 1/20 their original volume in a buffer of 25 mM Tris, 10 mM EDTA at pH 8.0 and containing lysozyme at 1 mg/ml. After a 30 minute incubation at room temperature the cells had lysed and were then further disrupted by sonication. The non-ionic detergent, Triton® X-100 was added to a final concentration of 2%, the cell lysate was mixed for 1 hour at room temperature and then centrifuged at 10,000 xg. The insoluble pellet fraction after these steps was saved. The major protein component of this fraction had a Mr of about 40 kDa as judged by Commassie blue staining of samples after SDS-gel electrophoresis. This same protein component was recognized in Western blot analysis by swine and mouse antisera raised against the authentic 39 kDa T. hyo protein obtained from the UP2 fraction. This recombinant protein was also recognized in immunoblots probed with sera from pigs that had recovered naturally from swine dysentery.

The 39kDa recombinant antigen obtained in this Example closely resembles but is not identical to the 39 kDa antigen of the UP2 fraction of T. hyo.; however, they have common epitopes recognized in a single sera.

12

Example 3 - 60 kDa antigen

Cells were grown, harvested, and initially processed as described in Example 1.

The detergent solubilized pool was centrifuged at 100,000 x g for 90 min and the supernatant fraction was applied to a column of DEAE-Sephacel® ion exchange chromatography resin. The protein was bound to the column and the excess Tween-20 detergent was washed through with 25 mM Tris bufffer pH 6.8, 0.1% Tween, 10 mM NaCl. The protein was then rapidly eluted with 1 M NaCl in the Tris/Tween buffer. The protein peak was collected, dialyzed against the low salt buffer and then rechromatographed on DEAE Sephacel® again. This time the protein was eluted with a two component linear gradient of 10mM to 300 mM NaCl and 0 to 2% Zwittergent 3-12 (Cal-Biochem) in the Tris-Tween buffer. Samples were analyzed by SDS-gel electrophoresis and fractions containing the 60 kDa protein were pooled. The protein was precipitated with 9 volumes of cold acetone, collected by centrifugation, and resolubilized by boiling in 1% SDS, 14 mM 2-mercaptoethanol in 25 mM Tris buffer pH 6.8, 5 mM EDTA. The soluble protein was then fractionated by molecular sieve chromatography on a column of Sepharose® 6B. Fractions containing the 60 kDa antigen were pooled, concentrated by acetone precipitation and brought to a concentration of 250 $\mu$g/ml in 1% SDS. It should be noted that there are a set of 3 proteins present in this preparation which differ only slightly in molecular weight (less than 2000) and all of which react with sera from pigs recovered from swine dysentery.

Immunization of a mouse and rabbit

A brown CB6-F1 mouse was immunized with 12 $\mu$g of the 60 kDa antigen pool described above. The protein was diluted into 100 $\mu$l of water, mixed with 100 $\mu$l of Freund's complete adjuvant mixed extensively and injected intraperitoneally. After two weeks the mouse was injected with 12 $\mu$g of antigen in Freund's incomplete adjuvant. Serum was obtained and used in an immunoblot assay to demonstrate its specificity towards the 60 kDa antigen. A New Zealand white rabbit was vaccinated twice with 25 $\mu$g of the 60 kDa antigen using Freund's complete and then incomplete adjuvants at an interval of 3 weeks. Serum obtained from the rabbit was also used in an immunoblot assay.

Construction of Genomic Library

A genomic library was constructed as in Example 2.

Identification of a recombinant clone by immunoscreening (uses method of Young, R.A. and Davis, R.W. (1983) PNAS 80 1194).

A set of agar plates was made which contained E. coli strain LE392 in top layer agar mixed with an aliquot of bacteriophage lambda gt11 which carries the T. hyo genomic library. The phage plaques were transferred to nitrocellulose and then reacted with a solution containing the mouse 60-2 sera diluted 1 in 1000 in phosphate buffered saline buffer containing 0.05% Tween®-20 (PBST). The filters were then washed with 1st antibody buffer (10mM Tris pH 7.2, 0.9% NaCl, 0.5% sodium azide, and 3% bovine serum albumin fraction V) and then reacted with an iodinated sheep-anti-mouse second antibody labeled with I[125]. (Amersham). The filters were washed in a second antibody buffer (Tris buffered saline as above, no azide, and 1% gelatin instead of BSA) and then placed against Kodak® X-ray film and positive clones (phage plaques which had produced protein recognized by the mouse serum against the 60 kDa antigen) were observed as exposed spots. The film spots were then aligned with the original agar plate so that the phage in that clone were picked and recultured (plaque purified) twice more to get a pure phage stock of clones. The purified recombinant phage were then replated on E. coli and the plaques were retested against both the mouse 60-2 sera and rabbit anti 60 kDa sera. Both sera gave a strong positive reaction by immunoblot using a peroxidase conjugated second antibody and a color producing reaction (reagents from Vector Labs).

The phage was then lysogenized into E. coli strain Y1089 and a cell lysate of this recombinant strain was analyzed by gel electrophoresis followed by transfer to nitrocellulose (Western blot). Reaction with the mouse 60-2 serum showed an immunoreactive band at the size of E. coli beta-galactosidase (120 kDa) as expected if the coding sequence of a portion of the T. hyo gene had been fused to that of the galactosidase gene present in the lambda phage vector.

A phage lysate was prepared to obtain the DNA of the recombinant clone and a 2 kbp EcoR1 fragment was isolated and recloned into plasmid vector pUC 19 to yield plasmid pTREP 101. Plasmid DNA was prepared by standard methods and the nucleotide sequence of the terminal regions of the cloned segment was determined. An open reading frame across the EcoR1 site and into the T. hyo DNA segment was found which matched that of the galactosidase in the phage vector and also showed the presence of the EcoR1 linker attached to the Alul sites of the T. hyo DNA (consistent with the method of preparation of the genomic library). This open reading frame of T. hyo DNA codes for a 10 kDa peptide and terminates with a TGA stop codon, which is likely to be the C-terminal segment of the 60 kDa protein of T. hyo.

A unique HindIII site was found 41bp downstream from the EcoR1 site in the cloned segment. The plasmid pTREP101 was digested with HindIII which liberated a 1700bp fragment which was then cloned into the unique HindIII site of expression plasmid pWHA93 to allow fusion of the T. hyo open reading frame with that of prochymosin in the pWHA93 vector. This resulting plasmid, pTREP 103, (Fig. 4) was found to direct the synthesis of large amounts of an insoluble protein in E. coli which contains prochymosin fused to the T. hyo protein segment. The expression and recovery of protein was effected as described with respect to the 39 kDa protein. This protein was shown to be 50kDa (40kDa prochymosin + 10kDa T. hyo protein segment) by gel electrophoresis. This insoluble protein is recognized by the mouse and rabbit sera raised against the 60kDa protein purified from T. hyo in immunoblots. The pTREP103 product is also recognized by sera from pigs recovered from swine dysentery.

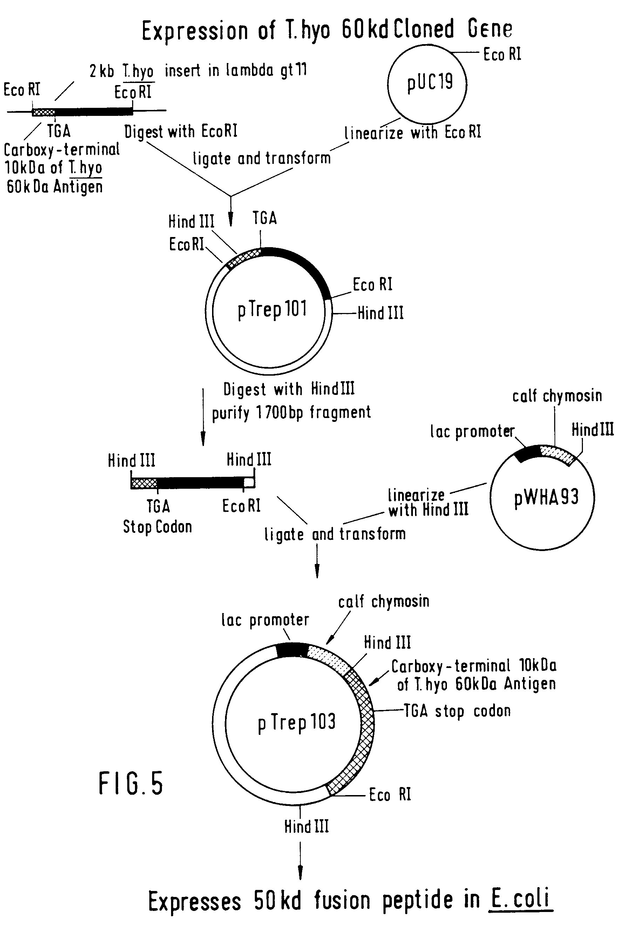

The sequence for preparing pTREP103 is schematically shown in Figure 5.

Example 4

TEST OF SUBUNIT VACCINE MATERIALS IN ANIMAL TRIALS

Test conditions:

Pigs weighed 40 lbs when first vaccinated, and were revaccinated 5 weeks after the first injection. Vaccine volume of 2 mls was mixed with 0.5 ml of a mineral oil adjuvant and administered by intramuscular injection.

Animals were infected with cultures of T. hyo 3 weeks after the second vaccination by oral inoculation of a slurry of broth and agar grown organims. Each animal received $10^9$ organisms.

| Vaccine antigen | Each dose | #Sick Pigs #Total | Days to onset of disease* | Weight gain from 5 wks pre-challenge to 7 wks post-challenge |
|---|---|---|---|---|
| controls (non-vaccinated | —— | 5/5 | 13(4) | 96 lbs |
| pTrep 9 recombinant fusion protein (38 kDa) purified and solubilized in 0.5% SDS (Example 1) | 1 mg | 4/5 | 22(12) | 99 lbs (3.1% more than control) |
| pTrep 103 recombinant fusion protein (60 kDa) purified and solubilized in 0.5% SDS (Example 3) | 1 mg | 5/5 | 20(8) | 108 lbs (12.5% more than control) |

*mean value (standard deviation)

Although the invention has been particularly described with respect to using the expressed protein for producing a vaccine, it is to be understood that the expressed protein may also be employed as a diagnostic for determining. T. hyo antibody. As a further alternative, the expressed protein may be employed to elicit antibodies and the elicited antibodies employed as a vaccine.

14

**Claims**

**Claims for the following Contracting States : AT, BE, CH, DE, FR, GB, GR, IT, LU, NL, SE**

1. An expression vehicle including the DNA sequence shown in Figure 3 or a fragment thereof which encodes a protein which is capable of eliciting an antibody which recognises the 38 kDa T. hyo. antigen.

2. An expression vehicle including the DNA sequence shown in Figure 6 or a fragment thereof which encodes a protein which is capable of eliciting an antibody which recognises the 39 kDa T. hyo. antigen.

3. An expression vehicle including the DNA sequence shown in Figure 4 or a fragment thereof which encodes a protein which is capable of eliciting an antibody which recognises the 60 kDa T. hyo. antigen.

4. A host transformed with the expression vehicle of any of claims 1 to 3.

**Claims for the following Contracting State : ES**

1. A process for producing a transformed host, which comprises transforming the host with an expression vehicle including the DNA sequence shown in Figure 3 or a fragment thereof which encodes a protein which is capable of eliciting an antibody which recognizes the 38 kDa T. hyo. antigen.

2. A process for producing a transformed host, which comprises transforming the host with an expression vehicle including the DNA sequence shown in Figure 6 or a fragment thereof which encodes a protein which is capable of eliciting an antibody which recognizes the 39 kDa T. hyo. antigen.

3. A process for producing a transformed host, which comprises transforming the host with an expression vehicle including the DNA sequence shown in Figure 4 or a fragment thereof which encodes a protein which is capable of eliciting an antibody which recognizes the 60 kDa T. hyo. antigen.

**Patentansprüche**

**Patentansprüche für folgende Vertragsstaaten : AT, BE, CH, DE, FR, GB, GR, IT, LU, NL, SE**

1. Expressionsvehikel, umfassend die in Figur 3 dargestellte DNA-Sequenz oder ein Fragment derselben mit Kodierung für ein Protein mit der Fähigkeit zur Bildung eines das 38 kDa T. hyo.-Antigen erkennenden Antikörpers.

2. Expressionsvehikel, umfassend die in Figur 6 dargestellte DNA-Sequenz oder ein Fragment derselben mit Kodierung für ein Protein mit der Fähigkeit zur Bildung eines das 39 kDa T. hyo.-Antigen erkennenden Antikörpers.

3. Expressionsvehikel, umfassend die in Figur 4 dargestellte DNA-Sequenz oder ein Fragment derselben mit Kodierung für ein Protein mit der Fähigkeit zur Bildung eines das 60 kDa T. hyo.-Antigen erkennenden Antikörpers.

4. Mit dem Expressionsvehikel nach einem der Ansprüche 1 bis 3 transformierter Wirt.

**Patentansprüche für folgenden Vertragsstaat : ES**

1. Verfahren zur Herstellung eines transformierten Wirts, umfassend die Transformation des Wirts mit einem Expressionsvehikel, das die in Figur 3 abgebildete DNA-Sequenz oder ein Fragment derselben mit Kodierung für ein Protein mit der Fähigkeit zur Bildung eines das 38 kDa T. hyo.-Antigen erkennenden Antikörpers umfaßt.

2. Verfahren zur Herstellung eines transformierten Wirts, umfassend die Transformation des Wirts mit einem Expressionsvehikel, das die in Figur 6 dargestellte DNA-Sequenz oder ein Fragment derselben mit Kodierung für ein Protein mit der Fähigkeit zur Bildung eines das 39 kDa T. hyo.-Antigen erkennenden Antikörpers umfaßt.

3. Verfahren zur Herstellung eines transformierten Wirts, umfassend die Transformation des Wirts mit einem Expressionsvehikel, das die in Figur 4 dargestellte DNA-Sequenz oder ein Fragment derselben mit Kodierung für ein Protein mit der Fähigkeit zur Bildung eines das 60 kDa T. hyo.-Antigen erkennenden Antikörpers umfaßt.

**Revendications**
**Revendications pour les Etats contractants suivants : AT, BE, CH, DE, FR, GB, GR, IT, LU, NL, SE**

1. Véhicule d'expression comprenant la séquence d'ADN représentée sur la figure 3 ou un de ses fragments qui code pour une protéine qui est capable d'engendrer un anticorps qui reconnaît l'antigène de 38 kDa de Treponema hyodysenteriae.

2. Véhicule d'expression comprenant la séquence d'ADN représentée sur la figure 6 ou un ses fragments qui code pour une protéine qui est capable d'engendrer un anticorps qui reconnaît l'antigène de 39 kDa de Treponema hyodysenteriae.

3. Véhicule d'expression comprenant la séquence d'ADN représentée sur la figure 4 ou un de ses fragments qui code pour une protéine qui est capable d'engendrer un anticorps qui reconnaît l'antigène de 60 kDa de Treponema hyodysenteriae.

4. Hôte transformé avec le véhicule d'expression suivant l'une quelconque des revendications 1 à 3.

**Revendications pour l'Etat contractant suivant : ES**

1. Procédé de production d'un hôte transformé, qui comprend la transformation de l'hôte avec un véhicule d'expression comprenant la séquence d'ADN représentée sur la figure 3 ou un de ses fragments qui code pour une protéine qui est capable d'engendrer un anticorps qui reconnaît l'antigène de 38 kDa de Treponema hyodysenteriae.

2. Procédé de production d'un hôte transformé, qui comprend la transformation de l'hôte avec un véhicule d'expression comprenant la séquence d'ADN représentée sur la figure 6 ou un de ses fragments qui code pour une protéine qui est capable d'engendrer un anticorps qui reconnaît l'antigène de 39 kDa de Treponema hyodysenteriae.

3. Procédé de production d'un hôte transformé, qui comprend la transformation de l'hôte avec un véhicule d'expression comprenant la séquence d'ADN représentée sur la figure 4 ou un de ses fragments qui code pour une protéine qui est capable d'engendrer un anticorps qui reconnaît l'antigène de 60 kDa de Treponema hyodysenteriae.

pTrep 2

Bam HI
Sal I
Hind III

Nde I
Bgl II

Eco RI

Hpa I
Bgl II

Bgl II
Eco RI

EcoRV

Hind III

kDa

/////////////// = DNA encoding the 38kDa gene

———— = DNA from Treponema hyodysenteriae strain B204

................. = pUC9 DNA

MetVal IlleAsn AsnAsnIle.................Gly AlaLeuArgLeuLeuGln0
CATGGAGGAACCATATGGTTATCAACAATAATATA      GGTGCTCTTAGATTATTACAAT
GTACCTCCTTGGTATACCAATAGTTGTTATTATAT      CCACGAGAATCTAATAATGTTA
            NDE1

FIG. 1

17

# CONSTRUCTION OF 38 KDa FUSION EXPRESSION

FIG. 2

```
1   AAGCTTGGGTGGACATCTATAATTTTCTTAATAAAGCATCAAAAAAAATTTATCAAAAATC
    TTCGAACCCACCTGTAGATATTAAAAGAATTATTTCGTAGTTTTTTTTAAATAGTTTTTAG
    ^
    ┌─────────────┐
    │ 1 HIND111, │
    └─────────────┘

62  TCAAAGAAGATAAAAGATATAAATGGCGTGGAATTAAAATAGATTTAAAACCTATTTACA
    AGTTTCTTCTATTTTCTATATTTACCGCACCTTAATTTTATCTAAATTTTGGATAAATGT
                                              ^
    105 AHA111 DRAI,

122 CACCCCACTCTTTTATCTTTATTACTTTATTTTTTAATCATAACTTTTATTTGTTTTATA
    GTGGGGTGAGAAAATAGAAATAATGAAATAAAAAATTAGTATTGAAAATAAACAAAATAT

182 AATAAATTAGAAAAAGCATCCCCGCCCAAGTTTTTATTATAAATATAAATTTATTAAGCG
    TTATTTAATCTTTTTCGTAGGGGCGGGTTCAAAAATAATATTTATATTTAAATAATTCGC

242 CACGGTGAATACATCATTAAAGAGGGAATAAATAAAAAAATATAATTTTTTATTTACATA
    GTGCCACTTATGTAGTAATTTCTCCCTTATTTATTTTTTTATATTAAAAAATAAATGTAT

302 GTAATACTCTGGATGCGTAATTAAAATAAATTTATAATAGATAATATATATAAGAAGCTA
    CATTATGAGACCTACGCATTAATTTTATTTAAATATTATCTATTATATATATTCTTCGAT

362 AGTTATTTTAGATTAACATAATAGTATTAACATAATATTAGGGTATATTTTTTTATAAAA
    TCAATAAAATCTAATTGTATTATCATAATTGTATTATAATCCCATATAAAAAAATATTTT
                                              ^
    395 SSP1,

422 CTGAAGAAAAAATTTTTATTTAACTAAAAAAAAATTAATAATTAAAAAGCGATAACCGA
    GACTTCTTTTTTTAAAAATAAATTGATTTTTTTTTAATTATTAATTTTTCGCTATTGGCT

482 TAATAAATGTGTGTTCAGGGAAGAATACATACAAATAATTCATGGAGGAACCAT
    ATTATTTACACACAAGTCCCTTCTTATGTATGTTATTAAGTACCTCCTTGGTA
                                                     ^
                                          MetVal   ┐
                                          ATGGTT   │ N-terminal sequence
                                          TACCAA   │

    533 NDE1,
    ────────────────────────────────────────────────────

    IleAsnAsnAsnIleSerAlaIleAsnAlaGlnArgThrLeuLysPheArgGln
542 ATCAACAATAATATAAGTGCTATAAACGCACAACGCACTTTAAAATTCCGCCAA
    TAGTTGTTATTATATTCACGATATTTGCGTGTTGCGTGAAATTTTAAGGCGGTT
                                              ^
                                          ValAsp
                                          GTAGAT
                                          CATCTA
                                          ^^

    580 AHA111 DRAI, 598 BGLII, 599 DAM.METH,
    ────────────────────────────────────────────────────
    LeuLysLysAspSerAlaMetIleSerSerGlyMetArgIleAsnGlnAlaGly
602 CTTAAAAAAGATTCTGCTATGATTTCTAGCGGTATGAGAATCAATCAAGCTGGA
    GAATTTTTTCTAAGACGATACTAAAGATCGCCATACTCTTAGTTAGTTCGACCT

                                          AspAsp
                                          GATGAT
                                          CTACTA
```

FIGURE 3 (sheet 1)

19

```
            ┌────────────────────────────────────────────┐
     AlaSerGlyLeuAlaValSerGluLysMetArgThrGlnIleArgGlyLeuArg  ⎫
662  GCTTCTGGTTTAGCAGTTTCTGAGAAAATGAGAACTCAGATTCGTGGTTTACGT   ⎪
     CGAAGACCAAATCGTCAAAGACTCTTTTACTCTTGAGTCTAAGCACCAAATGCA   ⎪  peptide 1 sequence
                                                      ──────→  ⎪
                                                      MetAla   ⎪
                                                      ATGGCT   ⎪
                                                      TACCGA   ⎪
                                                               ⎪
     711 SNABI,                                                ⎪
     └─────────────────────────────────────────────────────  ⎭
     GluArgAsnThrGlnAspGlyIleSerPheIleGlnThrThrGluGlyTyrLeu
722  GAAAGAAATACTCAAGACGGTATATCTTTCATTCAAACTACTGAAGGATACTTA
     CTTTCTTTATGAGTTCTGCCATATAGAAAGTAAGTTTGATGACTTCCTATGAAT  ──────→
                                                      GluGlu
                                                      GAAGAA
                                                      CTTCTT

     ┌──┐
     ThrThrAsnIleLeuGlnArgIleArgGluLeuAlaIleGlnAlaAlaAsnGly
782  ACTACTAACATTCTTCAAAGAATTCGTGAATTAGCTATACAAGCTGCTAACGGT
     TGATGATTGTAAGAAGTTTCTTAAGCACTTAATCGATATGTTCGACGATTGCCA
                        ∧
                                                      IleTyr
                                                      ATCTAT
                                                      TAGATA

     801 ECOR1,

     ThrAspGluAspArgLeuTyrValGlnIleGluValSerGlnLeuValAspGlu
842  ACTGACGAAGATAGACTTTATGTACAAATCGAAGTTTCTCAGTTAGTAGACGAA
     TGACTGCTTCTATCTGAAATACATGTTTAGCTTCAAAGAGTCAATCATCTGCTT
                                                      IleAsp
                                                      ATCGAC
                                                      TAGCTG

            ┌───────────────────────────────────────────┐
     ArgValAlaSerGlnAlaGlnPheAsnLysLeuAsnMetLeuThrGlyArgPhe  ⎫
902  AGAGTTGCTTCACAAGCTCAATTCAACAAACTTAACATGTTAACAGGAAGATTC   ⎪
     TCTCAACGAAGTGTTCGAGTTAAGTTGTTTGAATTGTACAATTGTCCTTCTAAG   ⎪ peptide 2 sequence
                                     ∧                         ⎪
                                                      ──────→  ⎪
                                                      AlaArg   ⎪
                                                      GCTAGA   ⎪
                                                      CGATCT   ⎪
                                                       ∧∧      ⎪
     940 HPA1, 959 BGLII, 960 DAM.METH,                        ⎪
     ┌─────────────────────────────────────────────────────  ⎪
     SerThrGlyGluAsnThrProThrAlaSerMetTrpLeuHisIleGlyAlaAsn   ⎪
962  TCTACAGGTGAAAACACTCCTACAGCTTCTATGTGGTTACACATCGGTGCTAAT   ⎪
     AGATGTCCACTTTTGTGAGGATGTCGAAGATACACCAATGTGTAGCCACGATTA   ⎪
                                                      ──────→  ⎪
                                                      MetAsp   ⎪
                                                      ATGGAC   ⎪
                                                      TACCTG   ⎭
     ┌────────────┐ ──────── ────────────
     GluArgLysArgValTyrIleGlyThrMetAsnSerGlnAlaLeuGlyLeuLys
1022 GAAAGAAAACGTGTTTATATCGGTACTATGAACAGCCAAGCTCTTGGACTTAAA
     CTTTCTTTTGCACAAATATAGCCATGATACTTGTCGGTTCGAGAACCTGAATTT
                        ∧
                                                      AsnPro
                                                      AACCCA
                                                      TTGGGT
```

FIGURE 3 (sheet 2)

20

1057 BSTXI,

```
ValGlyProAlaValThrAlaThrPheIleSerValSerSerProAlaLysAlaAsnSer
GTAGGACCTGCTGTTACAGCTACATTTATCAGTGTTTCTAGCCCAGCTAAAGCTAACTCT
CATCCTGGACGACAATGTCGATGTAAATAGTCACAAAGATCGGGTCGATTTCGATTGAGA
```

```
ValIleGlyMetValAspGluAlaLeuLeuLysValLeuLysGlnArgSerAspLeuGly
GTTATCGGTATGGTAGACGAAGCTCTTCTTAAAGTATTAAAACAAAGATCTGACTTAGGT
CAATAGCCATACCATCTGCTTCGAGAAGAATTTCATAATTTTGTTTCTAGACTGAATCCA
```

1160 ASP700 XMN1, 1187 BGLII, 1188 DAM.METH,

```
AlaTyrGlnAsnArgLeuGluMetThrAlaGlnGlyLeuMetValGlyPheGluAsnMet
GCTTATCAGAACAGATTAGAAATGACAGCTCAAGGCTTAATGGTTGGATTCGAAAATATG
CGAATAGTCTTGTCTAATCTTTACTGTCGAGTTCCGAATTACCAACCTAAGCTTTTATAC
```

1250 ASU11 fsp2,

```
GlnAlaSerGluSerArgIleArgAspThrAspMetAlaGluAlaSerValLysLeuAla
CAGGCTTCTGAAAGCAGAATTCGTGATACAGATATGGCTGAAGCATCAGTTAAACTTGCT
GTCCGAAGACTTTCGTCTTAAGCACTATGTCTATACCGACTTCGTAGTCAATTTGAACGA
```

1278 ECOR1,

```
LysAspGlnIleLeuAsnGlnAlaAsnLeuSerMetLeuAlaGlnAlaAsnGlnLeuPro
AAAGATCAAATTCTTAACCAAGCTAACTTGTCTATGCTTGCTCAAGCTAATCAGTTACCA
TTTCTAGTTTAAGAATTGGTTCGATTGAACAGATACGAACGAGTTCGATTAGTCAATGGT
```

1325 DAM.METH, 1380 DRA3,

```
GlnGlyAlaLeuArgLeuLeuGlnOC
CAAGGTGCTCTTAGATTATTACAATAATTAATATTCAATTAGTTTTGTAATATTAACCAT
GTTCCACGAGAATCTAATAATGTTATTAATTATAAGTTAATCAAAACATTATAATTGGTA
```

1411 SSP1, 1430 SSP1,

```
ATTATAAAGGGGGACATTCTTAATTGAATGCCTCCCCTTTTTATTTTTAAACTTCTTATA
TAATATTTCCCCCTGTAAGAATTAACTTACGGAGGGGAAAAATAAAAATTTGAAGAATAT
```

1487 AHA111 DRAI,

```
AAATTTCAATAATAATATTAATCATATATTTATTACCATATTAAACTGCTTTGATATC
TTTAAAGTTATTATTATAATTAGTATATAAATAATGGTATAATTTGACGAAACTATAG
```

1515 SSP1, 1554 ECORV,

FIGURE 3 (sheet 3)

21

Sequence is LINEAR.

```
                  pWHA93 prochymosin------> |---Trep 60kDa ----->
                                            HISALASerLeuLysAsnValSer
  1                                         ...cgAAGCTTGAAAAATGTGAGT
                                                 ^

  1 ECOR1,   42 HIND111,


    ValLysSerArgValTyrTrpAlaGlyIlePheGlyGlyAspIleGlnAsnAsnAlaTrp
 61 GTAAAAAGTAGAGTATATTGGGCAGGAATTTTTGGAGGCGATATTCAAAATAATGCTTGG

    PheThrTyrThrIleSerGlyThrAlaTyrAsnAsnGluMetGlySerGlyValLeuGln
121 TTTACATATACAATATCAGGAACCGCTTATAATAATGAAATGGGAAGCGGAGTTTTACAG

    AsnIleGluAsnLeuAspProArgSerGluSerThrThrGlyMetIleTyrLeuSerGlu
181 AATATAGAAAACTTAGACCCTAGATCTGAATCTACTACAGGTATGATATACTTAAGTGAA
                             ^                              ^

202 BGLII,  231 AFL11,

    ThrLysAspIleSerValGlyGlyGlnLeuThrSerLeuLeuLysAM AM
241 ACAAAAGATATATCTGTTGGAGGTCAGTTAACTTCTCTTCTCAAATAGTAGCTGAGGTAA
                           ^

267 HPA1,

301 GGAGTATAGAAA......1400 nucleotides....AGgaattc
```

FIGURE 4

# Expression of T.hyo 60kd Cloned Gene

FIG.5

Expresses 50kd fusion peptide in _E.coli_

Nucleotide sequence of T. hyo gene insert of pTrep106

```
(linker) I--T. hyo DNA -->
1  GAATTCC CTATTTTATATTTTTAGTATAATAAAAAATATAAACTTCAATTTAGGTATGTATA
   CTTAAGG GATAAAATATAAAAATCATATTATTTTTTATATTTGAAGTTAAATCCATACATAT

   1 ECOR1


      (Signal peptide sequence -->
      MetLysLysPhePheLeuIleMetThrValLeuLeuSerMetSerTyrCysSerIlePhe
63    ATGAAAAAGTTTTTTCTAATTATGACAGTATTATTAAGTATGTCATATTGTTCAATTTTT
      TACTTTTTCAAAAAAGATTAATACTGTCATAATAATTCATACAGTATAACAAGTTAAAAA


         I--begin mature protein -->
      GlyMetTyrGlyAspGlnAspAspTrpIleAspPheLeuThrAspGlyAsnGlnPheArg
123   GGTATGTATGGAGATCAGGACGATTGGATTGATTTTCTTACAGACGGCAATCAGTTTAGA
      CCATACATACCTCTAGTCCTGCTAACCTAACTAAAAGAATGTCTGCCGTTAGTCAAATCT


      AlaArgMetAspGlnLeuGlyPheValLeuGlyAsnSerThrIleLysGlyThrPheGly
183   GCTAGAATGGATCAATTAGGATTTGTTTTAGGTAATAGTACTATTAAAGGTACTTTCGGT
      CGATCTTACCTAGTTAATCCTAAACAAAATCCATTATCATGATAATTTCCATGAAAGCCA


      PheArgThrGlnSerSerSerThrGlnLeuGlyTyrIleLeuLeuAsnAsnAsnLeuGly
243   TTTAGAACTCAAAGTTCATCAACTCAATTAGGATATATTTTGTTGAATAATAATCTTGGT
      AAATCTTGAGTTTCAAGTAGTTGAGTTAATCCTATATAAAACAACTTATTATTAGAACCA


      ThrTyrLeuGlyAlaThrIleSerGlyGlyIleGlyTyrThrSerGluAlaPheSerIle
303   ACTTATTTGGGAGCAACTATTTCTGGCGGTATAGGATATACTTCTGAGGCTTTTAGTATA
      TGAATAAACCCTCGTTGATAAAGACCGCCATATCCTATATGAAGACTCCGAAAATCATAT


      GlyIleGlyTyrAsnTyrThrSerHisSerLeuPheProThrSerAspAspPheGlySer
363   GGCATAGGCTATAATTATACCAGCCATTCCTTATTTCCTACTAGCGATAACTTTGGTTCT
      CCGTATCCGATATTAATATGGTCGGTAAGGAATAAAGGATGATCGCTATTGAAACCAAGA


      HisThrProValLeuMetIleAsnAlaLeuAsnAspAsnLeuArgIleValIleProVal
423   CATACTCCAGTACTTATGATTAATGCTTTAAATGATAATTTGAGGATAGTTATTCCTGTG
      GTATGAGGTCATGAATACTAATTACGAAATTTACTATTAAACTCCTATCAATAAGGACAC


      GlnIleLeuValHisAsnGluSerIleAspGlnLeuGlyTyrTyrArgAspAsnTyrLeu
483   CAAATATTAGTACATAATGAAAGTATTGATCAACTTGGTTACTATAGAGATAATTATTTA
      GTTTATAATCATGTATTACTTTCATAACTAGTTGAACCAATGATATCTCTATTAATAAAT


      GlyIleSerThrAspThrGlnIleArgTyrTyrThrGlyIleAspAlaPheAsnGluIle
543   GGTATAAGTACTGATACGCAAATAAGATATTATACAGGCATAGATGCTTTTAATGAAATA
      CCATATTCATGACTATGCGTTTATTCTATAATATGTCCGTATCTACGAAAATTACTTTAT


      ArgLeuTyrValLysTyrGlyGlnLeuGlyTyrLysIleAsnProHisAspThrIleAsn
603   AGATTATATGTAAAATATGGGCAATTAGGATATAAAATTAATCCGCATGATACTATAAAT
      TCTAATATACATTTTATACCCGTTAATCCTATATTTAATTAGGCGTACTATGATATTTA


      TyrThrGlnGluValLeuAlaArgSerPheGlyPheGluThrArgPheTyrPheLeuAsn
663   TATACACAAGAAGTTTTAGCAAGATCATTTGGTTTTGAAACAAGATTCTATTTTTTGAAT
      ATATGTGTTCTTCAAAATCGTTCTAGTAAACCAAAACTTTGTTCTAAGATAAAAAACTTA
```

FIGURE 6 (sheet 1)

```
     ThrAlaValGlyAsnValThrIleAsnProPheIleLysValAlaTyrAsnThrAlaLeu
723  ACTGCTGTTGGAAATGTAACTATCAATCCTTTTATTAAAGTAGCATATAATACAGCTTTG
     TGACGACAACCTTTACATTGATAGTTAGGAAAATAATTTCATCGTATATTATGTCGAAAC

     HisGlyTyrSerThrMetValArgAlaLeuAspGlyMetTyrGluGluIleGluGlyTyr
783  CATGGATATAGTACCATGGTAAGAGCATTGGATGGTATGTATGAAGAAATAGAAGGTTAT
     GTACCTATATCATGGTACCATTCTCGTAACCTACCATACATACTTCTTTATCTTCCAATA

     TyrProAspSerProAlaGlnSerTyrGluAspIleAsnValLysTrpAspLysAsnPro
843  TATCCAGATAGTCCTGCTCAATCATATGAAGATATTAATGTTAAATGGGATAAGAATCCT
     ATAGGTCTATCAGGACGAGTTAGTATACTTCTATAATTACAATTTACCCTATTCTTAGGA

     TyrAspValThrValGlnAlaValLeuGlyValThrAlaAsnSerAspIleValSerLeu
903  TATGATGTAACTGTGCAGGCAGTATTGGGAGTAACTGCTAATAGCGATATAGTATCACTT
     ATACTACATTGACACGTCCGTCATAACCCTCATTGACGATTATCGCTATATCATAGTGAA

     TyrValGluProSerLeuGlyTyrArgAlaLysTyrLeuGlyLysLeuThrTyrGluAsp
963  TATGTTGAGCCTTCTTTAGGTTATAGGGCTAAATATTTAGGAAAATTAACATATGAAGAT
     ATACAACTCGGAAGAAATCCAATATCCCGATTTATAAATCCTTTTAATTGTATACTTCTA

     ProAspGlyLysValAsnPheAspPheLysValAsnHisTyrLeuSerTrpGlyAlaTyr
1023 CCAGATGGAAAAGTTAATTTTGATTTTAAAGTTAATCATTATTTATCTTGGGGTGCTTAT
     GGTCTACCTTTTCAATTAAAACTAAAATTTCAATTAGTAATAAATAGAACCCCACGAATA

     AlaGluLeuTyrIleThrProValLysAspLeuGluTrpTyrPheGluMetAspValAsn
1083 GCAGAGCTTTATATAACACCGGTAAAAGATTTAGAATGGTATTTTGAAATGGATGTTAAT
     CGTCTCGAAATATATTGTGGCCATTTTCTAAATCTTACCATAAAACTTTACCTACAATTA

     AsnSerAspSerAspSerThrGlyIleProValSerPheAlaSerThrThrGlyIleThr
1143 AATAGTGATTCAGATTCTACAGGTATACCTGTTAGTTTTGCTTCTACTACAGGAATAACT
     TTATCACTAAGTCTAAGATGTCCATATGGACAATCAAAACGAAGATGATGTCCTTATTGA

     TrpTyrLeuProGluPheOC
1203 TGGTATTTGCCAGAATTTTAATTATAAAGCAAATTTTATATGATAAAATAAAAAATGTGG
     ACCATAAACGGTCTTAAAATTAATATTTCGTTTAAAATATACTATTTTATTTTTTACACC

1263 GGTATTTATTATTAAAAAATAAATACCCCACATTTTATTAAATAATTTTTTCAGTAATTT
     CCATAAATAATAATTTTTTATTTATGGGGTGTAAAATAATTTATTAAAAAAGTCATTAAA

                      (begin gene encoding a related protein-->
                                                            Met
1323 TACATTTATATATTTTTTAGTATAATAAAAATATAAACTTAAATTTAGGTATATACAATG
     ATGTAAATATATAAAAAATCATATTATTTTTATATTTGAATTTAAATCCATATATGTTAC

     LysLysIlePheLeuIleMetThrValLeuLeuSerMetSerTyrCysSerIlePheGly
1383 AAAAAAATTTTTCTAATTATGACAGTATTATTAAGTATGTCATATTGTTCAATATTTGGT
     TTTTTTTAAAAAGATTAATACTGTCATAATAATTCATACAGTATAACAAGTTATAAACCA

     MetTyrGlyAspGlnAspAspTrpIleAspPheLeuIleAspGlyAsnGlnPheArg
1443 ATGTATGGAGATCAGGACGATTGGATTGATTTTCTTATAGACGGTAATCAGTTTAGAG
     TACATACCTCTAGTCCTGCTAACCTAACTAAAAGAATATCTGCCATTAGTCAAATCTC

                                             (linker)

                                          GGAATTC
                                          CCTTAAG

                                          1502 ECOR1
               FIGURE 6 (sheet 2)
```

25

Partial DNA sequence of expression plasmid pTrep301

```
(plasmid pUC9-->        ¦<-- E. coli lacZ ------------->¦<-T. hyo
                        MetThrMetIleThrProSerLeuHisAlaSerTyrCys
  1 AATTTCACACAGGAAACAGCTATGACCATGATTACGCCAAGCTTGCATGCCTCATATTGT
    TTAAAGTGTGTCCTTTGTCGATACTGGTACTAATGCGGTTCGAACGTACGGAGTATAACA
                                           ^ 39 HIND111.

    signal ---->¦<--mature T. hyo 39 kDa protein-->
    SerIlePheGlyMetTyrGlyAspGlnAspAspTrpIleAspPheLeuThrAspGlyAsn
 61 TCAATTTTTGGTATGTATGGAGATCAGGACGATTGGATTGATTTTCTTACAGACGGCAAT
    AGTTAAAAACCATACATACCTCTAGTCCTGCTAACCTAACTAAAAGAATGTCTGCCGTTA

    GlnPheArgAlaArgMetAspGlnLeuGlyPheValLeuGlyAsnSerThrIleLysGly...
121 CAGTTTAGAGCTAGAATGGATCAATTAGGATTTGTTTTAGGTAATAGTACTATTAAAGGT...
    GTCAAATCTCGATCTTACCTAGTTAATCCTAAACAAAATCCATTATCATGATAATTTCCA...
```

The first 25 amino acids of this recombinant fusion protein were determined on protein isolated from E. coli transformed with plasmid pTrep301. The protein was isolated as a Triton X-100 insoluble aggregate and was further purified by preparative SDS gel electrophoresis.

FIGURE 7

pTrep 301pUC9
4093bp

PVU11 57
Hind111 238
BCL1 655

ECOR1 1646
PVU11 1738
NAR1 1809
AATZ 2151

bgal

39k fusion

FIG. 8